# EUROPEAN PATENT SPECIFICATION

(11) **EP 3 417 744 B1**
(45) Date of publication and mention of the grant of the patent: **27.01.2021**
(21) Application number: 17752723.1
(22) Date of filing: 10.02.2017
(51) Int. Cl.: A47C 31/00

(54) **REST ASSEMBLY FOR RECOVERING THE GEOELECTRIC FIELD**
RASTANORDNUNG ZUR WIEDERHERSTELLUNG DES GEOELEKTRISCHEN FELDES
ENSEMBLE DE LITERIE POUR LA RÉCUPÉRATION DU CHAMP GÉOÉLECTRIQUE

(30) Priority: 19.02.2016 ES 201630190
(43) Date of publication of application: 26.12.2018
(73) Proprietor: Jiménez Castillo, Rosa, 18101 Belicena (Granada) (ES)
(72) Inventor: Jiménez Castillo, Rosa, 18101 Belicena (Granada) (ES)
(74) Representative: Sahuquillo Huerta, Jesús
(86) International application number: PCT/ES2017/070077
(87) International publication number: WO 2017/140931

(56) References cited:
- WO-A1-2008/087225
- KR-A- 20050 007 260
- US-A1- 2010 064 433
- US-A1- 2015 226 278
- DATABASE WPI Thomson Scientific, London, GB; AN 2009-L86176 XP055411474, & WO 2009/091345 A1 (ISTIKBAL MOBILYA SANAYI VE TIC ET AL.) 23 July 2009 (2009-07-23)
- ULTRACONFORT TEJIDO DE BAMBU, 4 October 2012 (2012-10-04), XP055574834, Retrieved from the Internet: URL:https://web.archive.org/web/2012102410 0844/ http://ultraconfort.es/contents/view/3/cal idad- y-tecnologias
- HIMALAYAN CRYSTAL SALTS PILLOW, 2 May 2012 (2012-05-02), XP055574877, Retrieved from the Internet: URL:https://web.archive.org/web/2012050210 3458/ http://www. natural -salt- lamps.com/ natural -pain-relief-therapy.html
- Anonymous: "Crystal Royal Mattresses. Platinum", Crystal Royal Mattresses, 19 July 2015 (2015-07-19), XP009512757, Retrieved from the Internet: URL:https://web.archive.org/web/2015071915 1116/http://www.crystalroyalmattresses.com /?page=mattresses-platinum
- "LX Himalayan Salt Spa Treatment Massage Table", HYMALAYAN SALT SPA, 17 October 2015 (2015-10-17), pages 1-6, XP009512766, Pacoima (California) Retrieved from the Internet: URL:https://web.archive.org//web/201510172 13529/http://www.spaandequipment.com/LX-Hi malayan-Salt-Spa-Treatment-Massage-Table.h tml
- DATABASE WPI Thomson Scientific, London, GB; AN 2014-K95031 XP055411481, & CN 203 538 932 U (WANG QIGUANG) 16 April 2014 (2014-04-16)
- GOFLOR COLCHON BAMBU, 23 February 2015 (2015-02-23), XP055574920, Retrieved from the Internet: URL:https://web.archive.org/web/2015022301 2556/ http:/lugotexsl.com/virtuemart/medical/ant i-alergia/anti-microbiano-bambu-100- natural -sanitario/colchon-bambu-tejido-100- natural

## Description

### OBJECT OF THE INVENTION

The invention, as stated in the heading of the present specification, relates to a rest assembly for recovering the geoelectric field which provides, to the function it is intended for, advantages and characteristics, which will be described in detail below which entail a novelty in the current state of the art.

More specifically, the object of the invention focusses on a bed formed from a mattress base and a mattress, covering the assembly of elements and covers it comprises, the structural configuration of which, based on specific materials and elements, has the purpose of providing a rest system which recovers the terrestrial electrostatic and electromagnetic field, correctly polarising the bed user's cells, avoiding a magnetic depolarisation and, with this, avoiding rapid oxidation and cell death in the body since, through the use of said materials, it emits negative ions, thus balancing the amount of positive ions in the atmosphere, making the rest assembly allow rest in a balanced and healthy environment, in addition to insulating the electromagnetic fields.

### FIELD OF APPLICATION OF THE INVENTION

The field of application of the present invention is included within the sector of the industry devoted to manufacturing rest furniture and accessories, particularly focussing on the field of mattress bases and mattresses.

### BACKGROUND OF THE INVENTION

As is known, our physical and mental health is influenced, among other things, by the correct ionisation of the environment in which we live. Today there are more sources of positive ions than in the past, which creates an electrical imbalance which considerably affects us. To understand this, it suffices to think how our feelings improve in a mountain climate or in the middle of a pine forest, places where a large quantity of negative ions are concentrated.

The varied human activities and electrical applications produce positive ions which cause the deterioration of our physical and emotional wellbeing. Example: electrical networks, heating, cooling systems, televisions, radios, transmitters, radars, computers, extractors, microwaves, mobile phones, cigarette smoke, etc.

The excess positive ions produced by chemical and electromagnetic pollution is the cause of stress (it stimulates the production of noradrenaline), it may produce insomnia, migraines, exhaustion, hypertension, depression and aggravate asthma and respiratory allergies. Healthy and electrically balanced air contains a proportion of 4 negative ions and 5 positive ions. However, a greater quantity of negative ions improves environmental quality, eliminates particles in suspension from the air (pollen, dust, mites, bacteria) and it has positive neurobiological effects; it induces relaxation and favours the secretion of melatonin (it improves the quality of sleep and cell regeneration).

It is demonstrated that negative ions are advantageous and have the following qualities: they produce improvements in allergies, headaches and migraine, they reduce the severity of asthma attacks, they can improve the immune system, increase productivity in work, strengthen concentration, increase lung capacity, and reduce the susceptibility to recurrent flu and colds.

The quality of the air we breathe is essential for our health and wellbeing. Not only does it keep us alive, it also allows us to think more clearly, make better use of the hours of sleep and improve our health. Studies demonstrate that we receive 56% of our energy from the air we breathe; i.e. it is more than the energy of water and food combined. Intoxication due to the excessive number of positive ions in the air can be considered a cause of weakness, anxiety, depression, insomnia and diseases that we suffer from.

In nature, negative ions are created by means of wind, sunlight, waterfalls, storms and rain. In fresh air there are up to 4,000 negative ions per cubic centimetre (the size of a sugar cube), whilst close to a waterfall there can be up to 10,000 negative ions. In contrast, it is calculated that the number of ions in large cities does not reach 100 per cubic centimetre. Scientific studies demonstrate that salt crystal rocks can increase the negative ion count in its surroundings to by 300%. When pink salt crystals are heated, the absorb moisture and contact with these particles causes the release of negative ions, functioning as an absolutely natural ioniser.

Ionisers for homes and offices emerged as a need against the invasion of harmful radiations, generated by electrical items in closed environments. Electrical energy flows between particles of different polarities, called ions. A negative ion is an electronically charged molecule composed of oxygen. A positive ion is a molecule which has lost its electrons in the process of atmospheric pollution.

Planet Earth is surrounded and protected by an electromagnetic field, on which all forms of life depend. Our brain waves work in resonance with said frequency pattern (7.83 hertz), named in homage to the researcher who documented their existence: Schuman. However, when we are in the proximity of certain electrical appliances (televisions, computers, mobile phones, etc.), we are affected by their electromagnetic radiation that ranges from 100 and 160 hertz.

Hence, we are typically exposed (and for long periods of time) to frequency patterns up to twenty times higher than normal. This overload has an influence on the delicate balance of the body's electromagnetic field, causing an abnormal functioning of cells. Different studies demonstrate that this dissonance gives rise to various symptoms such as anxiety, insomnia, lack of concentration, memory problems, etc. and also generates the increase in free radicals in the body, closely related to tumour formation.

Thus, the current pace of life and the different aggressive electromagnetic fields to which humans are exposed on a daily basis seriously damage the health.

It would therefore be desirable to be able to avoid these drawbacks, the essential object of the present invention being through the development of a rest system which, formed by the mattress base and mattress assembly with the different elements and materials it comprises, allows recovery of the electrostatic field and, consequently, improves users' health.

Furthermore, and as reference to the current state of the art, it can be indicated that although multiple types of bed are known manufactured in a large variety of materials, the existence is not known of any that has technical, structural and constitutive characteristic similar to those specifically presented herein, as claimed.

### EXPLANATION OF THE INVENTION

Thus, the rest assembly for recovery of the geoelectric field proposed by the invention is configured as a novelty within its field of application, since, according to its implementation and specifically, the aforementioned objectives are satisfactorily reached, the characterising details that distinguishes it being conveniently set down in the final claims that accompany the present description.

Specifically, what the invention proposes, as previously noted, is a bed, understood as the rest assembly formed by a mattress base and a mattress, and the set of elements and cover said mattress comprises, which is distinguished as it has a structural configuration based on specific elements and materials that recover the electrostatic field, polarising the user's cells as they emit negative ions that balance the amount of positive ions normally existing in the atmosphere.

Therefore, said rest assembly comprises a mattress base, preferably articulated, adaptable to the weight, stature and shape of each person, manufactured in steamed beech wood laminate, so that it maintains a good flexibility and varnished with 100% natural water-based varnish, so that it does not emit any harmful substances that can be breathed in or absorbed by the skin.

For its part, the mattress is formed from a core of latex, made from a 100% natural emulsion of the resin of the Hevea brasiliensis tree, whipped and baked in a furnace at 120 degrees, with no chemical additives, thus avoiding the inhalation or absorption of any chemical or toxic product, such as formaldehyde, carbon dioxide, etc., present in any mattress that is not 100% natural.

The core of said mattress also incorporates a sheet made of 100% natural steamed and pressed coconut fibre, of around 3 cm thick, in which central part of this sheet made of coconut fibre there is a layer of rocks of pink salt and white quartz.

Preferably, the sheet made of coconut fibre is positioned in the lower part of the mattress, with the aim of ionising the atmosphere, absorbing moisture and acting as anti-radiation element. For its part, the rocks of pink salt are considered to be efficient natural ionisers, which also provide other health benefits.

Likewise, the mattress of the rest assembly claimed also incorporates a bamboo fibre mesh, located between the core of latex and the described sheet made of coconut fibre, and it should be indicated that bamboo is the only natural antibacterial, antifungal (fungicide) and antistatic plant. Antimicrobial protection is an inherent characteristic of bamboo fibre. This does not allow microbes to grow in the fabric.

Finally, the mattress has a cover whose fabric is made in 70% cashmere and 30% organic cotton, having a filling which, at least covers the upper part of the mattress and has a thickness of around 4 cm, made in 100% natural wool. This cover also incorporates a fabric of organic graphite and silver and an aluminium plate connected to a no-return device which act as earth connections for recovering the geoelectric field necessary for correct polarisation of the body on a cellular level in addition to eliminating the artificial electromagnetic fields that exist in the environment.

Therefore, the rest assembly proposed by the invention helps the body defend itself and recover from the aggressions we do not see, from sources of free radicals and the consequences of stress, up to being particularly beneficial for some diseases such as Parkinson's or arthritis. The rest assembly acts by helping to recover the terrestrial geoelectric field and also eliminating static electricity in the body, helping to protect the natural production of melatonin during the night.

In this regard, the rest system works in various fields in benefit of health:
- It recovers the terrestrial geoelectric field.
- It discharges the electromagnetism that has been accumulating during the day.
- It maintains a stable moisture level, guaranteeing good oxygenation during the night, which avoids sleeping on damp, which may lead to arthritis or rheumatism.
- It improves the production of melatonin, a natural sleep inducer, which favours cell regeneration.
- It delays the natural aging process as it decreases a large part of the free radicals, the causes of aging of our body and enhancing the body's natural antioxidants.
- It improves oxygenation of the body.
- It increases immune system capacity.
- It reduces anxiety levels.
- Due to its comfort, anatomical adaptability and natural materials of its composition, it increases the quality and quantity of the hours of continuous sleep, favouring rest and wellbeing.
- It reduces excess pressure on specific points of the body which improves blood circulation.
- It facilitates correct posture, which helps avoid lumbago, scoliosis, sciatica and cervical pathologies.

In short, the healthy properties of the rest assembly make the place where one rests an 8-hour daily treatment which, among other benefits, can help prevent degenerative diseases such as Parkinson's, Alzheimer's, Cancer, Diabetes, and as it increases natural melatonin production, it reduces aging on a cellular level, improving the quality of sleep and rest, etc. Furthermore, the manufacturing components of the rest system have properties that are highly beneficial due to the use of noble woods, natural and ecological materials.

It should be indicated that said properties are reaffirmed by studies performed that confirm it.

In particular, the results provided by the rest assembly are endorsed by several scientific studies performed by renowned specialists in anti-aging, sleep phases, electromagnetic fields and immunological system, some of which are summarised below:
- Study "Control of oxidative stress under controlled rest conditions"
   The academics responsible for the research certify that the use of this rest system from youth will have important beneficial effects on the quality of life connected to aging. It demonstrates that an apparently unimportant system, or that the adequate importance is not given to, has important consequences in the medium to long-term which may mean the difference between a life with more or less quality and health. This study has been awarded the prize "Best general scientific research" in the 21^{st} Congress of the Spanish Society of Plastic Surgery and Anti-aging.
- Study "Rest system and immunological variations"
   Study performed by the Professor Mónica de la Fuente del Rey from the Faculty of Biological Sciences of the Complutense University of Madrid. External services.
   After 30 days of healthy rest in this system a rejuvenation is detected of some bodily functions such as adherence, which lowers preventing that the cells adhere to the endothelial cells and cannot reach their place of actions; chemotaxis, capacity of the cells to reach the place in the body where its defensive activity or recognition of what is foreign is performed, and phagocytosis, capacity of ingestion of the infectious agents.
- Study "Measurement of the static electric field, VLF (*Very Low Frequency*,) and ELF (*Extremely Low Frequency*,). Measurement of the static electric field, VLF and ELF. Measurement of radiofrequencies."
   Performed by the Professor Dr. José Luis Bardasano in the University of Medicine of Alcalá de Henares. It demonstrates a beneficial effect of recovery of the terrestrial magnetic field on the physiology of voluntary subjects.

The pineal gland, pineal body or epiphysis is a body which synchronises the release of a certain hormone with the light-dark phases. It is considered a neuroendocrine transducer and a «biological clock». In 1958, a team from Yale University, headed by Aaron B. Lerner, discovered that the gland releases the hormone melatonin. The pineal gland, or epiphysis, is a body of the size of a pine nut located right in the geometric centre of our brain. Its biological mission is the internal segregation that is related to the regulation of the wake and sleep cycles (circadian rhythms) and the processes of puberty in addition to being a powerful antioxidant and participating in the apoptosis of cancer cells in the thymus. Also, a recipient magnet, i.e. it is sensitive to magnetic fields and it transforms its waves into neurochemical stimuli.

The assembly of the invention is, therefore, a rest system where all today's environmental problems have been considered. Correcting them with natural materials so that the bed where we spend a third of our lives becomes a healthy place and helps maintain the quality of life over the years.

The described rest assembly for recovery of the geoelectric field thus represents an innovation of structural and constitutive characteristics unknown until know, reasons which, together with its practical use, give it sufficient grounds to obtain the privilege of exclusiveness applied for.

### DESCRIPTION OF THE DRAWINGS

To complement the description being made and in order to aid towards a better understanding of the characteristics of the invention, a plan is attached to the present specification wherein, with illustrative and non-limitative character, the following has been represented:
Figure number 1.- Shows a sectional, schematic elevational view of a portion of the mattress base and mattress forming the rest assembly object of the invention, observing the main parts and elements it comprises in addition to the configuration and layout thereof, and it must be indicated that to facilitate a better observation thereof, some of the proportions have been exaggerated.

### PREFERRED EMBODIMENT OF THE INVENTION

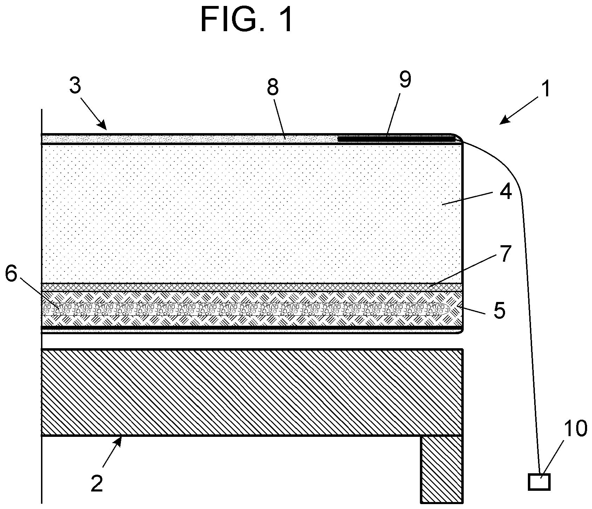

In light of the single described figure 1 and in accordance with the numbering adopted, a non-limitative example of the rest assembly for recovering the geoelectric field claimed can be observed, which comprises the parts and elements indicated and described in detail below.

So, relating to said figure 1, It can be observed how the rest assembly (1) in question, formed from a mattress base (2) and a latex mattress (3), is distinguished as it consists of a mattress base (2) manufactured in steamed beech laminate, varnished with 100% natural water-based varnish, and a mattress (3) formed from a core of latex (4), made from a 100% natural emulsion of the resin of the Hevea brasiliensis tree, whipped and baked in a furnace at 120 degrees, with no chemical additives, which also incorporates an anti-moisture ionising sheet (5) made of 100% natural steamed and pressed coconut fibre, and wherein there is a central layer (6) of rocks of pink salt and white quartz, and an antibacterial mesh (7) of bamboo fibre.

Preferably, the anti-moisture ionising sheet (5) made of coconut fibre is approximately 3 cm thick and is positioned in the lower part of the mattress, whilst the antibacterial mesh (7) of bamboo fibre, also preferably, is incorporated in the mattress (3) between the core of latex (4) and said anti-moisture ionising sheet (5) made of coconut fibre.

In any case, the mattress (3) also has a cover (8) made from fabric comprising a 70% cashmere and 30% organic cotton, with a filling which, at least, covers the upper part of the mattress with a thickness of around 4 cm, made in 100% natural wool.

Said cover (8) also incorporates a fabric of organic graphite and silver and an aluminium plate (9) connected to a device (10) acting as earth connection.

Having sufficiently described the nature of the present invention, as well as the manner of putting it into practice, it is not considered necessary to give further explanation in order for any person skilled in the art to understand its scope and the advantages derived therefrom.

## Claims

1. REST ASSEMBLY FOR RECOVERING THE GEOELECTRIC FIELD which, formed from a mattress base (2) and a latex mattress (3), consisting of a mattress base (2) manufactured in steamed beech wood laminate, varnished with 100% natural water-based varnish, and a mattress (3) formed from a core of latex (4), made from a 100% natural emulsion of the resin of the Hevea brasiliensis tree, whipped and baked in a furnace at 120 degrees, with no chemical additives, which, furthermore, incorporates an anti-moisture ionising sheet (5) made of 100% natural steamed and pressed coconut fibre, **characterized in that** it further comprises a central layer (6) of rocks of pink salt and white quartz, and an antibacterial mesh (7) of bamboo fibre; and **in that** said mattress (3) also has a cover (8) made with fabric comprising 70% cashmere and 30% organic cotton, with a filling which, at least, covers the upper part of the mattress with a thickness of around 4 cm, made in 100% natural wool which incorporates a fabric of organic graphite and silver and an aluminium plate (9) connected to a device (10) acting as earth connection.

2. REST ASSEMBLY FOR RECOVERING THE GEOELECTRIC FIELD, according to claim 1, **wherein** the anti-moisture ionising sheet (5) made of coconut fibre is approximately 3 cm thick.

3. REST ASSEMBLY FOR RECOVERING THE GEOELECTRIC FIELD, according to claim 1 or 2, **wherein** the anti-moisture ionising sheet (5) made of coconut fibre is positioned in the lower part of the mattress.

4. REST ASSEMBLY FOR RECOVERING THE GEOELECTRIC FIELD, according to claim 3, **wherein** the antibacterial mesh (7) of bamboo fibre is incorporated in the mattress (3) between the core of latex (4) and the anti-moisture ionising sheet (5) made of coconut fibre.

## Patentansprüche

1. REST MONTAGE ZUR WIEDERHERSTELLUNG DES GEOELEKTRISCHEN FELDES, das aus einem Matratzenboden (2) und einer Latexmatratze (3) besteht und aus einem Matratzenboden (2) besteht, der aus gedämpftem Buchenholzlaminat hergestellt und mit 100% natürlichem Lack auf Wasserbasis lackiert ist und eine Matratze (3) aus einem Latexkern (4), hergestellt aus einer 100% natürlichen Emulsion des Harzes des Hevea brasiliensis-Baumes, geschlagen und in einem Ofen bei 120 Grad ohne chemische Zusätze gebacken, die enthält außerdem eine Anti-Feuchtigkeitslonisationsfolie (5) aus 100% natürlichen gedämpften und gepressten Kokosfasern, die **dadurch gekennzeichnet ist, dass** sie ferner eine zentrale Schicht (6) aus Steinen aus rosa Salz und weißem Quarz sowie ein antibakterielles Netz (7) umfasst. aus Bambusfaser; und darin hat die Matratze (3) auch einen Bezug (8) aus Stoff, der aus 70% Kaschmir und 30% Bio-Baumwolle besteht, mit einer Füllung, die mindestens den oberen Teil der Matratze mit einer Dicke von etwa 4 cm bedeckt, hergestellt aus 100% natürlicher Wolle, die einen Stoff aus organischem Graphit und Silber und eine Aluminiumplatte (9) enthält, die mit einer Vorrichtung (10) verbunden ist, die als Erdungsverbindung fungiert.

2. RESTANORDNUNG ZUR WIEDERHERSTELLUNG DES GEOELEKTRISCHEN FELDES nach Anspruch 1, wobei die Anti-Feuchtigkeits-Ionisationsfolie (5) aus Kokosfaser ungefähr 3 cm dick ist.

3. RESTANORDNUNG ZUR WIEDERHERSTELLUNG DES GEOELEKTRISCHEN FELDES nach Anspruch 1 oder 2, wobei die feuchtigkeitsfeindliche ionisierende Folie (5) aus Kokosfaser im unteren Teil der Matratze angeordnet ist.

4. RESTANORDNUNG ZUR WIEDERHERSTELLUNG DES GEOELEKTRISCHEN FELDES nach Anspruch 3, wobei das antibakterielle Netz (7) aus Bambusfasern in die Matratze (3) zwischen dem Kern des Latex (4) und der Anti-Feuchtigkeitslonisationsfolie (3) eingearbeitet ist 5) aus Kokosfaser.

## Revendications

1. ENSEMBLE REPOSE POUR RECUPERER LE CHAMP GEOELECTRIQUE qui, formé d'un sommier (2) et d'un matelas en latex (3), constitué d'un sommier (2) fabriqué en bois de hêtre stratifié à la vapeur, verni avec un vernis 100% naturel à l'eau , et un matelas (3) formé à partir d'un noyau de latex (4), fabriqué à partir d'une émulsion 100% naturelle de la résine de l'arbre Hevea brasiliensis, fouetté et cuit dans un four à 120 degrés, sans additifs chimiques, qui, en outre, incorpore une feuille ionisante anti-humidité (5) faite de fibre de coco 100% naturelle cuite à la vapeur et pressée, **caractérisée en ce qu'**elle comprend en outre une couche centrale (6) de roches de sel rose et de quartz blanc, et une maille antibactérienne (7) de fibre de bambou; et **en ce que** ledit matelas (3) a également une housse (8) en tissu comprenant 70% de cachemire et 30% de coton biologique, avec un rembourrage qui, au moins, recouvre la partie supérieure du matelas d'une épaisseur d'environ 4 cm. , fabriqué à 100% de laine naturelle qui incorpore un tissu de graphite organique et d'argent et une plaque d'aluminium (9) reliée à un dispositif (10) faisant office de mise à la terre.

2. ENSEMBLE DE REPOSE POUR RECUPERER LE CHAMP GEOELECTRIQUE, selon la revendication 1, dans lequel la feuille ionisante anti-humidité (5) en fibre de coco a une épaisseur d'environ 3 cm.

3. ENSEMBLE DE REPOSE POUR RECUPERER LE CHAMP GEOELECTRIQUE, selon la revendication 1 ou 2, dans lequel la feuille ionisante anti-humidité (5) en fibre de coco est positionnée en partie inférieure du matelas.

4. ENSEMBLE REPOSE POUR RECUPERER LE CHAMP GEOELECTRIQUE, selon la revendication 3, dans lequel la maille antibactérienne (7) en fibre de bambou est incorporée dans le matelas (3) entre l'âme de latex (4) et la feuille ionisante anti-humidité (5) en fibre de coco.
